(19) Europäisches Patentamt
European Patent Office
Office européen des brevets

(11) EP 1 333 790 B1

(12) **FASCICULE DE BREVET EUROPEEN**

(45) Date de publication et mention de la délivrance du brevet:
**16.08.2006 Bulletin 2006/33**

(21) Numéro de dépôt: **01983656.8**

(22) Date de dépôt: **30.10.2001**

(51) Int Cl.:
***A61K 8/00*** *(2006.01)* ***A61Q 1/02*** *(2006.01)*

(86) Numéro de dépôt international:
**PCT/FR2001/003370**

(87) Numéro de publication internationale:
**WO 2002/036083 (10.05.2002 Gazette 2002/19)**

# (54) COMPOSITION COSMETIQUE CONTENANT UN AGENT DE COLORATION PHOTOCHROME ET SON UTILISATION POUR LE MAQUILLAGE ET/OU LE SOIN DE LA PEAU ET/OU DES PHANERES

KOSMETISCHE ZUSAMMENSETZUNG ENTHALTEND EINEN PHOTOCHROMEN FARBSTOFF, DEREN VERWENDUNG ALS MAKE-UP UND/ODER ZUR PFLEGE DER HAUT UND/ODER DER HAUTANHANGSGEBILDE

COSMETIC COMPOSITION CONTAINING A PHOTOCHROMIC COLOURING AGENT AND ITS USE FOR SKIN AND/OR SKIN APPENDAGE MAKE-UP AND/OR CARE

(84) Etats contractants désignés:
**AT BE CH CY DE DK ES FI FR GB GR IE IT LI LU MC NL PT SE TR**

(30) Priorité: **31.10.2000 FR 0014027**

(43) Date de publication de la demande:
**13.08.2003 Bulletin 2003/33**

(73) Titulaire: **L'ORÉAL**
**75008 Paris (FR)**

(72) Inventeurs:
- **KOLODZIEJ, Richard**
**F-75116 Paris (FR)**
- **SOISTIER, Nicolas**
**F-94270 Kremlin Bicetre (FR)**
- **SIMON, Jean-Christophe**
**162-0842 TOKYO (JP)**

(74) Mandataire: **Catherine, Alain et al**
**Cabinet Harlé & Phélip**
**7, rue de Madrid**
**75008 Paris (FR)**

(56) Documents cités:
**EP-A- 0 998 901** **US-A- 5 176 905**
**US-A- 5 628 934** **US-A- 5 762 915**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention se rapporte à une composition liquide émulsionnée de soin, et/ou de traitement, et/ou de maquillage de la peau et/ou des masses kératiniques, contenant au moins deux phases distinctes, l'une comprenant des agents de coloration photochromes et l'autre comprenant des matières colorantes non photochromes, pour améliorer les propriétés photochromiques de l'agent de coloration photochrome.

Les compositions de maquillage connues sont constituées d'un véhicule approprié et de différents agents de coloration destinés à conférer une certaine couleur aux compositions, avant et/ou après leur application sur la peau.

La gamme d'agents de coloration actuellement utilisée par les cosméticiens est assez limitée. Ces agents sont principalement des pigments organiques, des laques, des pigments minéraux ou des pigments nacrés.

Les laques permettent d'obtenir des couleurs vives, mais sont pour la plupart instables à la lumière, à la température et au pH. Certaines présentent également l'inconvénient de tâcher la peau de manière disgracieuse après application, par dégorgement du colorant.

Les pigments minéraux, en particulier les oxydes minéraux, sont au contraire très stables, mais donnent des couleurs plutôt ternes et pâles.

Les pigments nacrés permettent d'obtenir des couleurs variées mais qui ne sont pas intenses, à effets irisés mais le plus souvent assez faibles.

Il a alors été proposé d'utiliser des agents de coloration photochromes dans les compositions de maquillage ou capillaires, de manière à obtenir des changements agréables et variables dans le "rendu couleurs " des maquillages de la peau et/ou des cheveux.

Un agent de coloration photochrome est un agent ayant la propriété de changer de couleur et/ou d'opacité lorsqu'il est éclairé par de la lumière ultraviolette en présence ou non de lumière visible, et de revenir à l'état initial lorsqu'il n'est plus éclairé par la lumière ultraviolette. Par couleur, on entend toute couleur du spectre visible.

De tels agents de coloration trouvent notamment une application particulièrement intéressante dans les compositions cosmétiques, en particulier dans les compositions de maquillage telles que les fonds de teint, les fards à joues ou à paupières, les mascaras, les eye-liners, les rouges à lèvres, les laques à lèvres et les vernis à ongles.

En effet, on a constaté que le "rendu maquillage" d'une peau maquillée avec une telle composition est différent selon que l'on se trouve en éclairage naturel ou en éclairage artificiel. Ainsi, un maquillage réalisé en éclairage artificiel paraîtra plus clair en lumière naturelle. A l'inverse, un maquillage réalisé à l'extérieur paraîtra plus sombre dans un endroit éclairé artificiellement. Le maquillage prend une couleur adaptée à l'environnement du consommateur.

Il est connu d'utiliser conjointement dans des compositions cosmétiques des agents de coloration photochromes et des matières colorantes non photochromes. Dans ces applications connues, les agents de coloration photochromes et les matières colorantes non photochromes sont contenues dans une même phase.

Ainsi, le brevet européen EP-359.909 décrit un fond de teint émulsionné comprenant un pigment photochrome et un pigment classique non photochrome. Il apparaît clairement, si l'on se réfère au mode de préparation, que le pigment photochrome et le pigment non photochrome sont contenus dans une seule et même phase.

D'autre part, la demande de brevet européen EP-898.952 décrit l'utilisation d'un conducteur ionique pour améliorer le photochromisme d'une composition, notamment cosmétique, comprenant un composé photochrome et éventuellement des matières colorantes non photochromes habituellement utilisées dans les compositions cosmétiques, telles que des pigments, des nacres, des charges ou des colorants organiques.

De même, le brevet américain US-5989573 propose l'utilisation d'un composant particulier pour améliorer le photochromisme d'une composition cosmétique contenant un composé photochrome et éventuellement des matières colorantes non photochromes, le composant particulier étant susceptible d'accaparer au moins un état vacant d'une bande d'énergie correspondant à une lacune électronique du composé photochrome.

Or, on a constaté, que lorsqu'on associe des agents de coloration photochromes et des matières colorantes non photochromes, ces dernières altèrent les propriétés photochromiques des agents de coloration photochromes.

L'invention a donc pour objet de fournir une composition cosmétique, en particulier pour le soin, et/ou le traitement, et/ou le maquillage de la peau et/ou des phanères, en particulier du visage et du corps humain, tels que les lèvres, les cheveux, les cils, les sourcils et les ongles, comprenant à la fois des agents de coloration photochromes et des matières colorantes non photochromes, qui possède des propriétés photochromes améliorées.

L'invention a également pour objet l'utilisation d'une composition telle que définie précédemment pour soigner et/ou maquiller et/ou protéger la peau et/ou les phanères du visage et du corps humain.

L'invention a encore pour objet l'utilisation d'une composition telle que définie précédemment pour préparer un onguent destiné à traiter et/ou protéger la peau et/ou les phanères du visage et du corps humain.

Les propriétés photochromiques d'un composé peuvent être caractérisées à l'aide des coordonnées trichromatiques (L, a et b) de la manière décrite dans les exemples. Ces coordonnées permettent notamment la détermination du paramètre $\Delta E_{15}$ qui servira, dans la présente demande, à caractériser le photochromisme des agents de coloration photochromes. D'une manière générale, plus le paramètre $\Delta E_{15}$ est élevé, plus le composé est susceptible de changer

de couleur après irradiation.

Ainsi donc, l'invention a pour objet d'améliorer le paramètre $\Delta E_{15}$ de la composition, c'est-à-dire d'obtenir le paramètre $\Delta E_{15}$ le plus élevé possible.

Plus précisément, les buts de la présente invention sont atteints en réalisant une composition cosmétique liquide, sous forme d'émulsion, comportant au moins deux phases distinctes et contenant au moins un agent de coloration photochrome et au moins une matière colorante non photochrome, caractérisée en ce que le (ou les) agent(s) de coloration photochrome(s) et la (ou les) matière(s) colorante(s) non photochrome(s) se trouvent dans des phases distinctes de l'émulsion.

Les agents de coloration photochromes et les matières colorantes non photochromes sont soit dissous, soit dispersés selon leur solubilité. Les agents de coloration photochromes sont généralement dissous s'ils sont solubles et les pigments non photochromes dispersés.

L'agent de coloration photochrome peut être choisi parmi tous les composés photochromes de l'art antérieur susceptibles d'être utilisés dans le domaine d'application envisagé

On peut notamment citer les composés photochromes minéraux, complexes et plus particulièrement les aluminosilicates dopés et les oxydes et hydrates d'oxydes métalliques.

Les aluminosilicates ont une structure de base qui consiste en une cage formée par des tétraèdres de $AlO_4$ et de $SiO_4$ liés ensemble par leurs atomes d'oxygène. Certains éléments chimiques peuvent être présents dans les cages ainsi formées. On appelle ces éléments, des «éléments dopants ». L'aluminosilicate dopé est donc un aluminosilicate qui comprend au moins un élément dopant. Ces éléments dopants peuvent être des anions halogénures tels que les anions chlorure, iodure, bromure ou fluorure, seul ou en mélange. Les éléments dopants peuvent également se présenter sous la forme des groupements soufre, sélénium, $SO_4^{2-}$, $WO_4^{2-}$ ou hydroxyle, ou encore sous la forme d'ions métalliques tels que les ions obtenus à partir du fer, chrome, manganèse, cobalt et/ou nickel. On peut également utiliser un mélange de ces différents éléments dopants.

Parmi les aluminosilicates dopés, on peut citer ceux décrits dans la demande EP-A-0.847.751. Ces composés ont de préférence une structure de type : $R_8Al_6Si_6O_{24}X_n$
dans laquelle :

- R représente un élément choisi parmi les éléments Na, K, Cs, Rb, Li, Ag ou Ca, de préférence R représentant Na; et
- X représente un ou plusieurs éléments dopants tels que définis ci-dessus,
- n étant de 1 à 5, et de préférence de 1 à 3.

On peut en particulier citer parmi ces composés, ceux de la famille des sodalites qui ont pour formule : $Na_8Al_6Si_6O_{24}X_2$,
dans laquelle $X_2$ représente un ou plusieurs anions halogènes et notamment $Cl_2$, ClBr, $I_2$ ou $Br_2$.

On peut également citer les composés photochromes minéraux choisis parmi les oxydes métalliques, les hydrates desdits oxydes et leurs complexes tels que ceux décrits dans les brevets. US-A-5.989.573 et EP-B1-0.359.909, et notamment les oxydes de titane, de niobium; de silicium, d'aluminium, de zinc, d'hafnium, de thorium, d'étain, de thallium, de zirconium, de béryllium, de cobalt, de calcium, de magnésium, de fer et leurs mélanges. Parmi ces oxydes métalliques, on peut notamment citer les oxydes de titane, aluminium, zinc, zirconium, calcium, magnésium, silicium et fer. Les oxydes et hydrates d'oxydes de titane, aluminium, zinc, zirconium, calcium et magnésium sont préférés. De manière encore plus prérérentielle, on utilisera le dioxyde de titane qui peut être rendu photochrome à l'aide d'un métal choisi parmi le fer, le chrome, le cuivre, le nickel, le manganèse, le cobalt, le molybdène, tel que ou sous forme de sel tel qu'un sulfate, un chlorate, un nitrate, un acétate.

L'agent de coloration photochrome peut être incorporé dans la composition cosmétique selon l'invention en une quantité aisément déterminable par l'homme du métier sur la base de ses connaissances générales, et qui peut représenter de 0,01 à 30 % en poids du poids total de la composition, de préférence de 1 à 16 % en poids.

La composition cosmétique selon l'invention contient également des matières colorantes non photochromes, choisies parmi les matières colorantes habituellement utilisées dans les compositions cosmétiques, notamment les pigments, les charges, les nacres, les colorants lipophiles, les colorants hydrophiles et leurs mélanges.

Par pigments, il faut comprendre des particules blanches ou colorées, minérales ou organiques, insolubles dans la phase hydrophile liquide, destinées à colorer et/ou opacifier la composition. Le plus souvent, de tels pigments sont monocolores.

Par charges, il faut comprendre des particules incolores ou blanches, minérales ou de synthèse, lamellaires ou non lamellaires, destinées à donner du corps ou de la rigidité à la composition, et/ou dé la douceur, de la matité et de l'uniformité au maquillage.

Par nacres, il faut comprendre des particules irisées, notamment produites par certains mollusques dans leur coquille ou bien synthétisées. Ces charges et nacres servent notamment à modifier la texture de la composition.

Les pigments peuvent être blancs ou colorés, minéraux et/ou organiques, de taille usuelle ou nanométrique

ou goniochromatiques.

Comme pigments minéraux utilisables selon l'invention, on peut citer les oxydes de titane, de zirconium ou de cérium, ainsi que les oxydes de zinc, de fer ou de chrome, le bleu ferrique, l'hydrate de chrome et leurs mélanges.Parmi les pigments organiques utilisables selon l'invention, on peut citer le noir de carbone, les outremers (polysulfures d'aluminosilicates), le pyrophosphate de manganèse, et certaines poudres métalliques telles que celles d'argent ou d'aluminium, les laques de baryum, strontium, zirconium, calcium, aluminium, les dicétopyrrolopyrrole (DDP) décrits dans les documents EP-A-542.669, EP-A-787.730, EP-A-787.731 et WO 96/08537, et leurs mélanges.

Comme pigments goniochromatiques interférentiels, on peut citer les pigments de type à cristaux liquides (CL), comme l'HELICONE® de WACKER décrits dans les documents EP-A-0.815.826 et EP-A-0.953.33, ou de type multicouches (MC), comme les SICOPEARL FANTASTICO® de BASF décrits dans le document EP-A-0.953.33.

Les pigments peuvent être présents dans la composition à raison de 0 à 5%, de préférence de 0,01 à 5% en poids par rapport au poids total de la composition.

Les charges utilisables selon la présente invention peuvent être choisies dans le groupe constitué par le talc, le stéarate de zinc, le mica, la silice, le kaolin, les poudres de Nylon (Orgasol notamment) et de polyéthylène, le Téflon®, l'amidon, le nitrure de bore, les microsphères de copolymères telles que l'Expance1® (Nobel Industrie), le Polytrap® (Dow Corning), les microsphères de polyméthylmétacrylate, les microbilles de résine de silicone (Tospear1® de Toshiba, par exemple), le carbonate de calcium précipité, le carbonate ou l'hydrocarbonate de magnésium, les savons métalliques dérivés d'acides organiques carboxyliques ayant de 8 à 22 atomes de carbone. Les charges peuvent être présentes à raison de 0 à 30%, de préférence de 0,5 à 15% en poids par rapport au poids total de la composition.

Parmi les nacres envisageables, on peut citer la nacre naturelle, le mica recouvert d'oxyde de titane, d'oxyde de fer, de pigment naturel ou d'oxychlorure de bisumth tels que le mica titane coloré, et leurs mélanges. Les nacres peuvent être présentes dans la composition à raison de 0 à 20%, de préférence de 0,01 à 15% en poids par rapport au poids total de la composition.

La composition cosmétique selon l'invention peut en outre comprendre un colorant, notamment un colorant organique naturel tel que le carmin de cochenille, et/ou un colorant de synthèse tel que les colorants halogéno-acides, azoïdes et antraquinoniques. On peut également citer des colorants minéraux tels que le sulfate de cuivre.

La composition selon l'invention se présente sous forme d'une émulsion, notamment une émulsion huile-dans-eau (H/E), eau-dans-huile (E/H), ou multiple, par exemple triple. De préférence, elle se présente sous forme d'émulsion huile-dans-eau (H/E) ou eau-dans huilé (E/H).

Généralement, l'une au moins des phases est une phase grasse comprenant au moins un solvant organique et l'autre phase est une phase aqueuse.

Selon un premier mode de réalisation de l'invention, l'agent de coloration photochrome se trouve dans la phase grasse, et la matière colorante non photochrome se trouve dans la phase aqueuse.

Selon un secon mode de réalisation, la matière colorante non photochrome se trouve dans la phase grasse, et l'agent de coloration photochrome se trouve dans la phase aqueuse.

La phase aqueuse peut comprendre de l'eau, une eau florale telle que l'eau de bleuet et/ou une eau minérale telle que l'eau de Vittel, les eaux du bassin de Vichy, l'eau d'Uriage, l'eau de la Roche Posay, l'eau de la Bourboule, l'eau d'Enghien-les-Bains, l'eau de Saint Gervais-les-Bains, l'eau d'Allevar-les-Bains, l'eau de Digne, l'eau de Lucas, l'eau de Maizières, l'eau de Neyrac-les Bains, l'eau de Lons-le-Saunier, les Eaux Bonnes, l'eau de Rocherfort, l'eau de Saint Christau, l'eau des Fumades et l'eau de Tercis-les-Bains.

Ladite phase aqueuse peut également comprendre de 0% à 25%, de préférence de 0,1 à 20% en poids par rapport au poids total de la phase aqueuse, d'un monoalcool inférieur en $C_2$-$C_6$ et/ou d'un polyol tel que le glycérol, le butylèneglycol, l'isoprène glycol, le propylèneglycol, le polyéthylèneglycol.

La phase grasse comprend un ou plusieurs solvants organiques cosmétiquement acceptables (tolérance, toxicologie et toucher acceptables). Ces solvants organiques peuvent représenter de 0% à 95 %, de préférence de 0,1 à 90% en poids par rapport au poids total de la phase grasse, et peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple les monoalcools inférieurs linéaires ou ramifiés ayant de 1 à 8 atomes de carbone comme l'éthanol, le propanol, le butanol, l'isopropanol, l'isobutanol; les polyéthylène glycols ayant de 6 à 80 oxydes d'éthylène ; les polyols tels que le propylène glycol, l'isoprène glycol, le butylène glycol, le glycérol, le sorbitol ; les mono- ou di-alkyle d'isosorbide dont les groupements alkyle ont de 1 à 5 atomes de carbone; les éthers de glycol comme le diéthylène glycol monométhyl ou mono-éthyl éther et les éthers de propylène glycol comme le dipropylène glycol méthyl éther.

Comme solvants organiques amphiphiles, on peut citer les polyols tels les dérivés de polypropylène glycol et d'acide gras, de polypropylène glycol et d'alcool gras comme le PPG-23 oléyl éther et le PPG-36 oléate.

Les solvants organiques lipophiles peuvent être volatiles ou non volatiles.

Comme solvants organiques lipophiles non volatiles, on peut citer par exemple les esters gras tels que l'adipate de diisopropyle, l'adipate de dioctyle, les benzoates d'alkyle, le myristate d'isopropyle, le palmitate d'isopropyle, le

stéarate de butyle, le laurate d'hexyle, l'isononanoate d'isononyle, le palmitate de 2-éthyl hexyle, le laurate de 2-hexyl-décyle, le palmitate de 2-octyl-décyle, le myristate de 2-octyl-dodécyle, le succinate de di-(éthyl-2-hexyle), le malate de diisostéaryle, le lactate de 2-octyl-dodécyle, le triisostéarate de glycérine, le triisostéarate de diglycérine.

Comme solvants organiques lipophiles volatiles, on peut citer à titre d'exemple :

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane,
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane ;
- les silicones volatiles linéaires ayant de 2 à 9 atomes de silicium. Il s'agit par exemple de l'hexaméthyldisoloxane, de l'hexylheptaméthyltrisiloxane ou de l'octylheptaméthyltrisoloxane.

On peut également citer les huiles volatiles hydrocarbonées, telles que les isoparaffines et notamment l'isododécane ; et des huiles fluorées comme celle commercialisée sous la dénomination GALDEN® (MONTEFLUOS).

La phase grasse peut également comprendre des corps gras liquides à 25°C tels que des huiles siliconées non volatiles, des huiles d'origine animale, végétale ou minérale, ou des huiles fluorées ou perfluorées.

Parmi les huiles siliconées non volatiles, on peut citer :

- les polyalkyl($C_1$-$C_{20}$) siloxanes, et notamment ceux à groupements terminaux triméthylsilyle, de préférence ceux dont la viscosité est inférieure à 0,06 $m^2/s$ parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines,
- les huiles de silicone phénylées, notamment celles de formule :

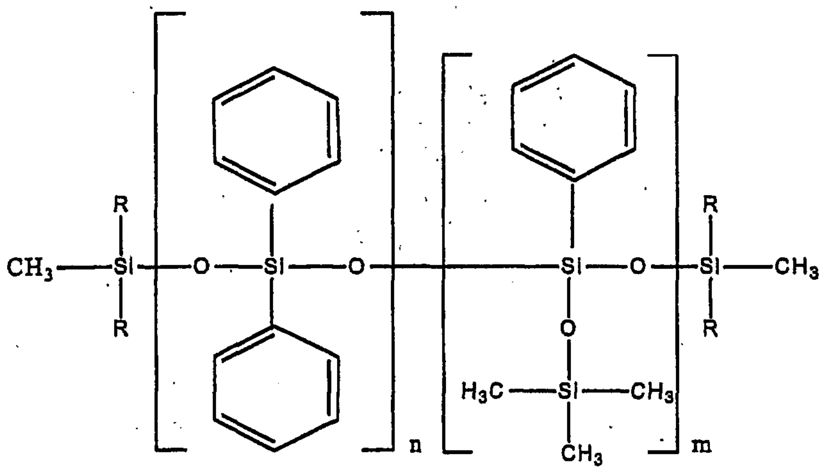

dans laquelle :

- R est un radical alkyle en $C_1$-$C_{30}$, un radical aryle ou un radical aralkyle,
- n est un nombre entier de 0 à 100, et
- m est un nombre entier de 0 et 100, sous réserve que la somme (m+n) est un nombre entier de 1 à 100.

Parmi les huiles d'origine animale, végétale ou minérale, et notamment les huiles animales ou végétales formées par des esters d'acide gras et de polyols, en particulier les triglycérides liquides, on peut citer :

- les huiles de tournesol, de maïs, de soja, de courge, de pépins de raisin, de sésame, de noisette, d'abricot, d'amandes ou d'avocat ; les huiles de poisson, le tricaprocaprylate de glycérol,
- les huiles végétales ou animales de formule $R^1COOR^2$, dans laquelle $R^1$ représente le reste d'un acide gras supérieur comportant de 7 à 19 atomes de carbone et $R^2$ représentant une chaîne hydrocarbonée ramifiée contenant de 3 à 20 atomes de carbone, par exemple, l'huile de Purcelin ; l'huile de paraffine, de vaseline, le perhydrosqualène, l'huile de germes de blé, de calophyllum, de sésame, de macadamia, de pépins de raisin, de colza, de coprah,

d'arachide, de palme, de ricin, de jojoba, d'olive ou de germes de céréales, les esters d'acides gras ; les alcools ; les acétylglycérides ; les octanoates, décanoates ou ricinoléates d'alcools ou de polyalcools ; les triglycérides d'acides gras ; les glycérides ;

La composition cosmétique selon l'invention peut comprendre en outre d'autres corps gras, qui peuvent être choisis par l'homme du métier sur la base de ses connaissances générales, de manière à conférer à la composition finale les propriétés souhaitées, par exemple en consistance et/ou en texture. Ces corps gras additionnels peuvent être des cires, des gommes et/ou des corps gras pâteux d'origine animale, végétale, minérale ou synthétique, ainsi que leurs mélanges.

On peut notamment citer :

- les gommes de silicones,
- les cires d'origine animale, végétale, minérale ou synthétique telles que les cires microcristallines, la paraffine, le pétrolatum, la vaseline, l'ozokérite, la cire de montan ; la cire d'abeilles, la lanoline et ses dérivés ; les cires de candellila, d'Ouricury, de Carnauba, du Japon, le beurre de cacao, les cires de fibres de lièges ou de canne à sucre ; les huiles hydrogénées concrètes à 25°C, les ozokérites, les esters gras et les glycérides concrets à 25°C ; les cires de polyéthylène et les cires obtenues par synthèse de Fischer-Tropsch ; les huiles hydrogénées concrètes à 25°C ; lés lanolines ; les esters gras concrets à 25°C ; les cires de silicone ; les cires fluorées.

La composition cosmétique selon l'invention peut éventuellement comprendre également au moins un tensioactif présent dans la composition à raison de 0 à 30%, de préférence de 0,1 à 25% en poids par rapport au poids total de la composition.

Ce sont principalement des dérivés anioniques, non ioniques, amphotères ou zwitterioniques.

On peut utiliser un tensioactif ou un mélange de tels tensioactifs.

Parmi les tensioactifs anioniques qui peuvent être utilisés seuls ou en mélange, on peut citer les acides carboxyliques et les sels d'acides carboxyliques. Les, acides carboxyliques pouvant être utilisés sont des acides gras, comportant une chaîne alkyle linéaire ou ramifiée, saturée ou insaturée, ayant de 6 à 16 atomes de carbone et de préférence 10 à 14 atomes de carbone. Les sels de tels acides gras constituent des savons. Comme sels, on peut utiliser par exemple les sels alcalins, les sels alcalino-terreux, les sels d'ammoniaque, les sels d'aminoalcools et les sels d'aminoacides, et notamment les sels de sodium, de potassium, de magnésium, de triéthanolamine, de N-méthylgiucamine, de lysine et d'arginine. L'acide carboxylique peut être en particulier l'acide laurique ou l'acide myristique.

Comme savon, :on peut citer par exemple les sels de potassium des acides gras en C10 à C14, et notamment le sel de potassium de l'acide laurique et le sel de potassium de l'acide myristique.

Comme autres tensioactifs anioniques pouvant être utilisés dans la composition de l'invention, on peut citer par exemple les acides carboxyliques éthoxylés et leurs sels ; les sarcosinates et acyl-sarcosinates et leurs sels tels que le lauroyl sarcosinate de sodium ; les taurates et méthyltaurates et leurs sels ; les iséthionates et acyl iséthionates, produits de réaction d'acides gras comportant de 10 à 22 atomes de carbone, avec l'acide isethionique, et leurs sels tels que l'iséthionate de sodium et le cocoyl-iséthionate de sodium ; les sulfosuccinates et leurs sels, les alkylsulfates et alkyléthersulfates et leurs sels, notamment le laurylsulfate de sodium ou de triéthanolamine, et le lauryléther sulfate de sodium ou de potassium ; les monoalkyl- et dialkylesters d'acides phosphorique et leurs sels, tels que par exemple le mono- et dilauryl phosphate de sodium, le mono- et dilauryl phosphate de potassium, le mono- et dilauryl phosphate de triéthanolamine, le mono- et dimyristyl phosphate de sodium, le mono- et dimyristyl phosphate de potassium, le mono- et dimyristyl phosphate de diéthanolamine, le mono- et dimyristyl phosphate de triéthanolamine; les alkane sulfonates et leurs sels ; les sels biliaires tels que les cholates, déoxycholates, taurocholates, taurodéoxycholates, les lipoaminoacides et leurs sels tels que les acylglutamates mono- et di-sodiques ; les tensioactifs géminés, bipolaires, tels que décrits dans Surfactant Sciences series, vol. 74 édité par Krister Homberg.

Parmi les tensioactifs non ioniques susceptibles d'être utilisés comme tensioactifs, on peut citer par exemple les éthers de polyols comportant des chaînes grasses (8 à 30 atomes de carbone), tels que les éthers gras de sorbitol ou de glycéryle oxyéthylénés ; les éthers et esters de polyglycérol ; les alcools gras polyoxyéthylénés qui sont par exemple des éthers formés d'unités d'oxyde d'éthylène et d'au moins une chaîne d'alcool gras ayant de 10 à 22 atomes de carbone. A titre d'exemple d'alcools gras polyoxyéthylénés, on peut citer les éthers d'alcool laurylique comportant plus de 7 groupes oxyéthylénés ; les alkyl polyglucosides dont le groupe alkyle comporte de 1 à 30 atomes de carbone, comme par exemple le décylglucoside, le laurylglucoside, le cétostéarylglucoside, le cocoyl-glucoside; les alkyl glucopyranosides et alkylthioglucopyranosides ; les alkyl maltosides ; les alcoyl N-méthylglucamide; les esters de : sorbitan polyoxyéthylénés qui comportent généralement de 1 à 100 unités d'éthylène glycol et de préférence de 2 à 40 unités d'oxyde d'éthylène (OE) ; les esters d'aminoalcools ; et leurs mélanges.

Comme tensioactifs amphotères ou zwitterioniques susceptibles d'être utilisés comme tensioactifs, on peut citer par exemple les bétaïnes tels que la diméthylbétaïne, la coco-bétaïne et la coco-amidopropylbétaïne; les sulfobétaïnes tels que la coco-amidopropylhydroxysultaïne ; les alkylamphoacétates tels que le cocoamphodiacétate ; et leurs mélanges.

La composition selon l'invention peut également comprendre de 0 à 10%, de préférence de 0,1 à 5% en poids par rapport au poids total de la composition, d'au moins un co-émulsionnant qui peut être choisi parmi le monostérarate de sorbitan oxyéthyléné, des alcools gras tels que l'alcool stéarylique ou l'alcool cétylique, ou les esters d'acides gras et de polyols tels que le stéarate de glycéryle.

La composition selon l'invention peut encore comprendre un ou plusieurs agents épaississants dans des concentrations préférentielles allant de 0 à 6%, de préférence de 0,1 à 5% en poids par rapport au poids total de la composition. L'agent épaississant peut être choisi parmi :

- les biopolymères polysaccharidiques comme la gomme de xanthane, la gomme de caroube, la gomme de guar, les alginates, les celluloses modifiées telles que l'hydroxyéthylcellulose, la méthylcellulose, l'hydroxypropylcellulose et la carboxyméthylcellulose, les dérivés de l'amidon, les dérivés d'éthers de cellulose possédant des groupes ammonium quaternaires, les polysaccharides cationiques ;
- les polymères synthétiques comme les acides polyacryliques tels que les polymères poly(méth)acrylates de glycéryl tels que l'HISPAGEL® ou le LUBRAGEL® des sociétés HISPANO QUIMICA ou GARDIAN, la polyvinylpyrrolidone, l'alcool polyvinylique, les polymères réticulés d'acrylamide et d'acrylate d'ammonium tels que le PAS 5161 ou BOZEPOL C® de HOECHST ; les copolymères acrylate/octylacrylamide tels que le Dermacryl® de National Starch ; les polymères à base de polyacrylamide tels que le SEPIGEL® 305 de SEPPIC, les polymères réticulés d'acrylamide et de chlorure de méthacryloyloxyéthyltriméthylméthylammonium tels que le SALCARE® SC 92 de ALLIED COLLOIDS,
- le silicate de magnésium et d'aluminium.

La composition cosmétique selon l'invention peut éventuellement comprendre un polymère filmifiable ou non. Ces polymères peuvent être dissous ou dispersés dans le milieu cosmétiquement acceptable. En particulier, ils peuvent être présents sous forme de solution dans un solvant organique ou sous forme de dispersion de particules de polymère.

Les compositions selon l'invention comprennent avantageusement une dispersion stable de particules généralement sphériques d'au moins un polymère dans une phase liquide physiologiquement acceptable Ces dispersions peuvent notamment se présenter sous forme de nanoparticules de polymères en dispersion stable dans ladite phase grasse. Les nanoparticules sont de préférence d'une taille comprise entre 5 et 600 nm, étant donné qu'au-delà d'environ 600 nm; les dispersions deviennent beaucoup moins stables.

Encore un avantage de la dispersion de polymère de la composition de l'invention est la possibilité de faire varier la température de transition (Tg) du polymère ou du système polymérique (polymère plus additif du type plastifiant), et de passer ainsi d'un polymère mou à un polymère plus ou moins dur, permettant de régler les propriétés mécaniques de la composition en fonction de l'application envisagée.

Il est possible d'utiliser des polymères, de préférence ayant une (Tg) basse ou égale à la température de la peau. On obtient ainsi une dispersion qui peut filmifier lorsqu'elle est appliquée sur un support, ce qui n'est pas le cas lorsque l'on utilise des dispersions de pigment minéraux selon l'art antérieur.

Les polymères utilisables dans la composition de l'invention ont de préférence un poids moléculaire de l'ordre de 2000 à 10 000 000 et une (Tg) de 100°C à 300°C.

Lorsque le polymère présente une température de transition vitreuse trop élevée pour l'application souhaitée, on peut lui associer un plastifiant usuellement utilisé dans le domaine d'application et notamment parmi les composés susceptibles d'être des solvants du polymère.

Parmi les polymères filmifiables, on peut citer des homopolymères ou des copolymères radicalaires, acryliques ou vinyliques, de préférence ayant une Tg inférieure à 40°C et notamment les acrylates de méthyle éventuellement copolymérisés avec l'acide acrylique et les dérivés de la cellulose tels que la nitrocellulose et l'acétobutyrate de cellulose.

Parmi les polymères non filmifiables, on peut citer des homopolymères ou copolymères radicalaires, vinyliques ou acryliques, éventuellement réticulés, ayant de préférence une Tg supérieure ou égale à 40°C, tels que le polyméthacrylate de méthyle, le polystyrène ou le polyacrylate de tertiobutyle.

De façon non limitatives, les polymères de l'invention peuvent être choisis parmi les polymères ou copolymères suivants: polyuréthannes, polyuréthannes acryliques, polyurées, polyurée-polyuréthannes, polyester-polyuréthannes, polyéther-polyuréthannes, polyesters, polyester-amides, polyesters à chaîne grasse alkydes: polymères ou copolymères acryliques et/ou vinyliques: copolymères, acryliques-silicone: polyacrylamides: polymères siliconés, polymères fluorés et leurs mélanges.

On citera en particulier les copolymères d'acide (méth)acrylique et d'au moins un monomère ester d'acide (méth)acrylique linéaire, ramifié ou cyclique et/ou d'au moins un monomère amide d'acide (méth)acrylique mono ou disubstitué linéaire, ramifié ou cyclique ; les copolymères acide (méth)acrylique/(méth)acrylate de tertio-butyle et/ou (méth) acrylate d'isobutyle/(méth)acrylate d'alkyle en $C_1$-$C_4$; les terpolymères acide (méth)acrylique/acrylate d'éthyle/méthacrylate de méthyle ; les tétrapolymères méthacrylate de méthyl/acrylate de butyle ou d'éthyle/acrylate ou méthacrylate d'hydroxyéthyle ou de 2-hydroxypropyle/acide (méth)acrylique ; les copolymères d'acide acrylique et de méthacrylate d'alkyle en $C_1$-$C_4$ ; les terpolymères de vinyl pyrrolidone, d'acide acrylique et de méthacrylate d'alkyle en $C_{1-20}$, les copolymères amphotères ; les esters vinyliques d'acide ramifiés ; les esters vinyliques d'acide benzoïque; les copolymères d'acide (méth)acrylique et d'au moins un monomère oléfinique ; les copolymères de monoacide vinylique et/ou de monoacide allylique.

Parmi les résines, on peut citer les résines du type aryl-sulfonamide formaldéhyde ou aryl-sulfonamide époxy ; les résines du type acrylique, styrénique, acrylate-styrénique et vinylique, et les résines siliconées.

La composition selon l'invention peut également comprendre au moins un plastifiant, tel que le phosphate de tricrésyle, le benzoate de benzyle, le citrate de triéthyle, le citrate de tributyle, l'acétylcitrate de triéthyle, l'acétylcitrate de triéthyl-2 hexyle, le camphre ; les éthers de glycol ; l'huile de ricin oxyéthylénée à 40 moles d'oxyde d' éthylène ; le propylène glycol ; le butyl glycol; l'éthylène glycol monométhyléther acétate; les éthers de propylène glycol ; les éthers esters de propylène glycol et d'éthylène glycol ; les esters de diacides tels que les phtalates et adipates de diéthyle, dibutyle et de diisopropyle, les tartrates de diéthyle et dibutyle, les succinates de diéthyle et de dibutyle, les sébaçates de diéthyle et de dibutyle, les phosphates de diéthyle, de dibutyle et de diéthyl-2 hexyle, l'acétyl citrate de diéthyle ou de dibutyle; les esters de glycérol. Les plastifiants peuvent être généralement présents à une teneur allant de 1 % à 40% en poids par rapport au poids total de la composition.

La composition selon l'invention peut, de plus, comprendre au moins un des ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, polymères synthétiques) ou grasse, les parfums, les actifs hydrophiles. (hydratants, par exemple choisis dans le groupe constitué par l'eau ou les alcools polyhydriques ayant de 2 à 8 atomes de carbone et de 2 à 6 fonctions hydroxy, par exemple choisis parmi l'éthylène glycol, la glycérine, le propanediol-1,2 la diglycérine, l'érythritol, l'arabitol, l'adonitol, le sorbitol, le dulcitol et le D-panthénol) ou lipophiles (par exemple choisi dans le groupe constitué par la lanoline et les filtres UVA ou B), les antioxydants, les colorants, les huiles essentielles, les extraits végétaux, les vitamines et leurs dérivés (telles que les vitamines A, B, C et E), les sphingolipides (céramides), les polymères liposolubles notamment les hydrocarbonés (tels que le polybutène, les polyalkylènes, les polyacrylates et les polymères siliconés compatibles avec les corps gras), et leurs mélanges.

Les quantités de ces différents ingrédients sont celles classiquement utilisées dans les domaines concernés et par exemple de 0 à 20%, de préférence de 0,01 à 15% en poids par rapport au poids total de la composition.

Bien entendu, l'homme du métier veillera à choisir les éventuels composés complémentaires et/ou leur quantité, de manière telle que les propriétés avantageuses du produit cosmétique selon l'invention ne soient pas, ou substantiellement pas, altérées par l'adjonction envisagée.

La composition selon l'invention peut être un produit de soin, et/ou de traitement, et/ou de maquillage de la peau. Les compositions selon l'invention trouvent une application particulière dans le domaine des fonds de teint et des crèmes de soin, teintées ou non.

Bien entendu, la composition selon l'invention doit être cosmétiquement acceptable, c'est-à-dire contenir un milieu physiologiquement acceptable, non toxique et susceptible d'être appliqué sur la peau des êtres humains.

L'invention est illustrée plus en détail dans les exemples suivants. Dans les exemples, sauf indication contraire, tous les pourcentages et parties sont exprimés en poids.

## EXEMPLES

### Méthode de détermination du paramètre $\Delta E$ à l'aide des coordonnées trichromatiques

Les propriétés photochromiques d'un composé donné (par exemple l'agent de coloration photochrome) peuvent être caractérisées de la manière suivante, à l' aide des coordonnées trichromatiques (L, a et b).

Le composé en poudre est tassé dans une coupelle métallique.

On mesure les coordonnées trichromatiques ($L_0$, $_0$, $b_0$) de la poudre tassée à l'aide d'un colorimètre « CM 2002 ».

On irradie la coupelle pendant 15 minutes à l'aide d'une lampe émettant dans la totalité du spectre solaire (lampe au Xénon, Suntest CPS de marque Héraeus, reproduisant le spectre solaire), puis l'on mesure les nouvelles coordonnées ($L_{15}$, $a_{15}$ et $b_{15}$) qui reflètent le changement de couleur suite à l'irradiation.

On détermine le paramètre $\Delta E_{15}$ de la manière suivante :

$$\Delta E_{15} = [(L_{15}-L_0)^2 + (a_{15} - a_0)^2 + (b_{15} - b_0)^2]^{1/2}$$

Pour l'ensemble des exemples, l'agent de coloration photochrome comprend l'oxyde de titane et de la silice dopé au fer, vendu par la société C.C.I.C. par l'intermédiaire d'IKEDA sous la dénomination "PHOTOGENICA® 4 ".

Pour que les comparaisons effectuées aient leur pleine signification, chaque fond de teint possède, dans des proportions identiques, les mêmes pigments minéraux non photochromes et le même agent de coloration photochrome (« PHOTOGEIVICA® 4»).

EXEMPLE 1

On prépare un premier fond de teint émulsionné FDT 1 selon l'invention, en mélangeant les ingrédients indiqués dans le tableau 1, comme suit :

- L'agent de coloration photochrome PHOTOGENICA® 4 est dissous dans un peu d'eau et de propylène glycol, à température ambiante. Tout le broyat pigmentaire est ensuite broyé à la tricylindre (3 passages).
- Les pigments minéraux non-photochromes (oxydes de fer jaune, rouge et noir) sont dissous dans un peu d'isononanoate d'isononyle, à température ambiante. Tout le broyat pigmentaire est ensuite broyé à la tricylindre (3 passages).
- Dans un bécher M1, on introduit le reste d'eau et on le porte à une température de 75°C. On commence l'agitation au Raynerie (modérée) et on ajoute le Veegum.
- On attend 10 minutes puis on additionne le polysorbate 20 et le reste de propylène glycol.
- On ajoute le broyat pigmentaire contenant l'agent, de coloration photochrome PHOTOGENICA®4 dans le bécher M1. On maintient l'agitation et la température.
- On additionne la charge.
- Dans un bécher M2, on chauffe le reste d'isononanoate d'isononyle à 75°C. On additionne l'acide stéarique et le méthyl glucose sesquistéarate, sous agitation au Raynerie.
- Après 15 minutes, on met le broyat pigmentaire contenant les pigments minéraux non-photochromes (oxydes de fer jaune, rouge et noir) dans le bécher M2.
- Lorsque ces pigments se sont bien dispersés, on commence l'émulsion en versant le contenu du bécher M2 dans le bécher M1, sous agitation vigoureuse au Moritz, et on laisse l'ensemble pendant 10 minutes.
- On diminue l'agitation et on ajoute la silicone. On attend 10 minutes et on ajoute l'eau.

EXEMPLE 2

On prépare un deuxième fond de teint FDT 2 selon l'invention, en mélangeant les ingrédients indiqués dans le tableau 1 comme suit :

- L'agent de coloration photochrome PHOTOGENICA® 4 est dissous dans un peu d'isononanoate d'isononyle, à température ambiante. Tout le broyat pigmentaire est ensuite broyé à la tricylindre (3 passages).
- Les pigments minéraux non-photochromes (oxydes de fer jaune, rouge et noir) sont dissous dans un peu d'eau et de propylène glycol, à température ambiante. Tout le broyat pigmentaire est ensuite broyé à la tricylindre (3 passages).
- Dans un bécher M1, on introduit le reste d'eau et on le porte à une température de 75°C. On commence l'agitation au Raynerie (modérée) et on ajoute le Veegum.
- On attend 10 minutes puis on additionne le polysorbate 20 et le reste de propylène glycol.
- On ajoute le broyat pigmentaire contenant les pigments minéraux non-photochromes dans le bécher M1. On maintient l'agitation et la température.
- On additionne la charge.
- Dans un bécher M2, on chauffe le reste d'isononanoate d'isononyle à 75°C. On additionne l'acide stéarique et le méthyl glucose sesquistéarate, sous agitation au Raynerie.
- Après 15 minutes, on met le broyat pigmentaire contenant l'agent de coloration photochrome PHOTOGENICA® 4, dans le bécher M2.
- Lorsque ce pigment s'est bien dispersé, on commence l'émulsion en versant le contenu du bécher M2 dans le bécher M1, sous agitation vigoureuse au Moritz, et on laisse l'ensemble pendant 10 minutes.
- On diminue l'agitation et on ajoute la silicone. On attend 10 minutes et on ajoute l'eau.

EXEMPLE COMPARATIF 3

On prépare un troisième fond de teint FDT 3, en mélangeant les ingrédients indiqués dans le tableau 1 comme suit :

- L'agent de coloration photochrome PHOTOGENICA® 4 et les pigments minéraux (oxydes de fer jaune, rouge et noir) non photochromes sont dissous dans un peu d'eau et de propylène glycol, à température ambiante. Tout le broyat pigmentaire est ensuite broyé à la tricylindre (3 passages).
- Dans un bécher appelé M1, on introduit le reste d'eau et on le porte à une température de 75°C. On commence l'agitation au Raynerie (modérée) et ajouter le Veegum.
- On attend 10 minutes puis on additionne le polysorbate 20 et le reste de propylène glycol.
- On ajoute le broyat pigmentaire contenant l'agent de coloration photochrome PHOTOGENICA® 4 et les pigments minéraux non photochromes dans le bécher M1. On maintient l'agitation et la température.
- On additionne la charge.
- Dans un bécher M2, on chauffe le reste d'isononanoate d'isononyle à 75°C. On additionne l'acide stéarique et le méthyl glucose sesquistéarate, sous agitation au Raynerie.
- Après 15 minutes, on commence l'émulsion en versant le contenu du bêcher M2 dans le bécher M1, sous agitation vigoureuse au Moritz, et on laisse pendant 10 minutes.
- On diminue l'agitation et on ajoute la silicone. On attend 10 minutes et on ajoute l'eau.

TABLEAU 1

| Composition | FDT1 (%) | FDT2 (%) | FDT3 (%) |
|---|---|---|---|
| Eau | 37.6 | 37.6 | 37.6 |
| Veegum | 0.7 | 0.7 | 0.7 |
| Propylène glycol | 6 | 6 | 6 |
| Polysorbate 20 | 3.7 | 3.7 | 3.7 |
| Oxyde de fer jaune | 0.22 | 0.22 | 0.22 |
| Oxyde de fer rouge | 0.12 | 0.12 | 0.12 |
| Oxyde de fer noir | 0.05 | 0.05 | 0.05 |
| Photogénica 4 | 8.61 | 8.61 | 8.61 |
| Nylon | 4.5 | 4.5 | 4.5 |
| Isononanoate d'isononyle | 21.7 | 21.7 | 21.7 |
| Acide stéarique | 1 1 | 1 | 1 |
| Méthyl glucose sesquistearate | 3.5 | 3.5 | 3.5 |
| Silicone | 10 | 10 | 10 |
| Eau | 2.3 | 2.3 | 2.3 |

On mesure les caractéristiques des mélanges selon la méthode ci-dessus. On obtient les résultats donnés dans le tableau 4 :

| | $\Delta E_{15}$ |
|---|---|
| FDT1 | 5,6 |
| FDT2 | 5,6 |
| FDT3 | 5,03 |

On constate donc, que pour une même proportion de pigments minéraux et d'agent de coloration photochrome, la séparation entre ces ingrédients permet d'obtenir une valeur du paramètre $\Delta E_{15}$ notablement plus élevée.

## Revendications

1. Composition cosmétique liquide, sous forme d'émulsion, comportant au moins deux phases distinctes et contenant au moins un agent de coloration photochrome et au moins une matière colorante non photochrome, **caractérisée en ce que** le (ou les) agent(s) de coloration photochrome(s) et la (ou les) matière(s) colorante(s) non photochrome (s) se trouvent dans des phases distinctes de l'émulsion.

2. Composition selon la revendication 1, **caractérisée en ce que** l'agent de coloration photochrome est choisi parmi les oxydes métalliques photochromes, les hydrates d'oxydes métalliques photochromes, les complexes des oxydes ou des hydrates d'oxydes métalliques photochromes, et les aminosilicates dopés.

3. Composition selon la revendication 2, **caractérisée en ce que** l'agent de coloration photochrome est un oxyde métallique photochrome choisi parmi les oxydes de titane, de niobium, de silicium, d'aluminium, de zinc, d'hafnium, de thorium, d'étain, de thallium, de zirconium, de béryllium, de cobalt, de calcium, de magnésium ou un hydrate de ces oxydes.

4. Composition selon l'une quelconque des revendications précédentes, **caractérisée en ce que** l'agent de coloration photochrome représente de 0,01 à 30% et de préférence de 1 à 16% en poids, par rapport au poids total de la composition.

5. Composition selon l'une quelconque des revendications précédentes, **caractérisée en ce que** la matière colorante non photochrome est choisie parmi les colorants lipophiles, les colorants hydrophiles, les pigments minéraux et organiques, les nacres et leurs mélanges.

6. Composition selon la revendication 5, **caractérisée en ce que** la matière colorante non photochrome est un pigment présent dans la composition à raison de 0 à 5%, de préférence de 0,01 à 5% en poids par rapport au poids total du produit.

7. Composition selon les revendication 5 ou 6, **caractérisée en ce** la matière colorante non photochrome est un pigment minéral choisi parmi l'oxyde de fer jaune, l'oxyde de fer rouge, l'oxyde de fer noir et leurs mélanges.

8. Composition cosmétique selon l'une quelconque des revendications précédentes, **caractérisée en ce qu'**elle se présente sous forme d'une émulsion eau-dans-huile (E/H), huile-dans-eau (H/E), ou multiple.

9. Composition selon l'une quelconque des revendications précédentes, **caractérisée en ce que** l'une au moins des phases est une phase grasse comprenant au moins un solvant organique, et l'autre phase est une phase aqueuse.

10. Composition cosmétique selon la revendication 9, **caractérisée en ce que** le solvant organique est choisi parmi les solvants organiques hydrophiles, les solvants organiques lipophiles, les solvants amphiphiles et leurs mélanges.

11. Composition cosmétique selon la revendication 10, **caractérisée en ce que** le solvant organique est l'isononanoate d'isononyle.

12. Composition cosmétique selon l'une quelconque des revendications 9 à 11, **caractérisée en ce que** l'agent de coloration photochrome se trouve dans la phase grasse, et la matière colorante non photochrome se trouve dans la phase aqueuse.

13. Composition cosmétique selon l'une quelconque des revendications 9 à 11, **caractérisée en ce que** la matière colorante non photochrome se trouve dans la phase grasse, et l'agent de coloration photochrome se trouve dans la phase aqueuse.

14. Composition selon l'une quelconque des revendications précédentes, **caractérisée en qu'**il contient au moins un additif choisi parmi les conservateurs, les épaississants de phase aqueuse ou grasse, les parfums, les actifs hydrophiles ou lipophiles, les antioxydants, les colorants, les huiles essentielles, les extraits végétaux, les vitamines et leurs dérivés, les sphingolipides, les polymères liposolubles notamment hydrocarbonés et leurs mélanges

15. Composition selon la revendication 14, **caractérisée en ce que** l'additif est choisi parmi les biopolymères polysaccharidiques, les polymères synthétiques, les hydratants, les filtres UVA ou B, les vitamines A, B, C et E, les céramides,

le polybutène, les polyalkylènes, les polyacrylates et les polymères siliconés compatibles avec les corps gras et les mélanges.

**16.** Composition selon l'une quelconque des revendications précédentes, **caractérisée en ce qu'**elle se présente sous forme d'un produit de soin, et/ou de traitement, et/ou de maquillage de la peau et/ou des phanères tels que les laques à lèvres, les brillants à lèvres, les fonds de teint fluides ou solides, les produits anti-cernes ou de contour des yeux, les eye liners, les mascaras, les ombres à paupières, les crèmes de soin, teintées ou non et les vernis à ongles.

**17.** Utilisation de la composition cosmétique telle que définie selon l'une quelconque des revendications 1 à 16 pour le maquillage de la peau et/ou des phanères du visage et du corps humain.

**18.** Utilisation cosmétique de la composition telle que définie selon l'une quelconque des revendications 1 à 16, pour soigner et/ou maquiller et/ou protéger la peau et/ou les phanères du visage et du corps humain.

**19.** Utilisation de la composition cosmétique telle que définie selon l'une quelconque des revendications 1 à 16 pour soigner et/ou maquiller et/ou protéger la peau et/ou les phanères du visage et du corps humain.

**20.** Utilisation de la composition cosmétique telle que définie selon l'une quelconque des revendications 1 à 16, pour la préparation d'un onguent destiné à traiter et/ou protéger la peau et/ou les phanères du visage et du corps humain.

**21.** Procédé de traitement cosmétique de la peau et/ou des phanères du visage et du corps humain consistant à appliquer sur la peau et/ou les phanères une composition telle que définie selon l'une quelconque des revendications. à 16.

**22.** Utilisation, dans une composition cosmétique ou pour la fabrication d'une composition dermatologique, colorée, d'un agent de coloration photochrome, et d'une matière colorante non photochrome compris dans une composition cosmétique telle que définie selon l'une quelconque des revendications 1 à 16, afin de donner un rendu maquillage adapté à l'environnement.

**23.** Procédé de maquillage de la peau et/ou des phanères du visage et du corps humain consistant à appliquer sur la peau et/ou les phanères une composition cosmétique telle que définie selon l'une quelconque des revendications 1 à 16.

## Claims

**1.** Liquid cosmetic composition in the form of an emulsion comprising at least two distinct phases and containing at least one photochromic colouring agent and at least one non-photochromic colouring matter, **characterized in that** the photochromic colouring agent(s) and the non-photochromic colouring matter(s) are located in separate phases of the emulsion.

**2.** Composition according to Claim 1, **characterized in that** the photochromic colouring agent is selected from the photochromic metal oxides, photochromic metal oxide hydrates, the complexes of the photochromic metal oxides or metal oxide hydrates and the doped aminosilicates,

**3.** Composition according to Claim 2, **characterized in that** the photochromic colouring agent is a photochromic metal oxide selected from the oxides of titanium, niobium, silicon, aluminium, zinc, hafnium, thorium, tin, thallium, zirconium, beryllium, cobalt, calcium, magnesium or a hydrate of these oxides.

**4.** Composition according to any one of the preceding Claims, **characterized in that** the photochromic colouring agent represents from 0.01 to 30% and preferably from 1 to 16% by weight based on the total weight of the composition.

**5.** Composition according to any one of the preceding Claims, **characterized in that** the non-photochromic colouring matter is selected from the lipophilic colorants, the hydrophilic colorants, the mineral and organic pigments, the pearlescent preparations and their mixtures.

**6.** Composition according to Claim 5, **characterized in that** the non-photochromic colouring matter is a pigment

present in the composition at a concentration of 0 to 5%, and preferably from 0.01 to 5% by weight based on the total weight of the product.

**7.** Composition according to Claims 5 or 6, **characterized in that** the non-photochromic colouring matter is a mineral pigment selected from yellow iron oxide, red iron oxide, black iron oxide and their mixtures.

**8.** Cosmetic composition according to any one of the preceding Claims, **characterized in that** it is available in the form of a water-in-oil (W/O) emulsion, an oil-in-water (O/W) emulsion, or multiple emulsion.

**9.** Composition according to any one of the preceding Claims, **characterized in that** at least one of the phases is a fatty phase containing at least one organic solvent, and the other phase is an aqueous phase

**10.** Cosmetic composition according to Claim 9, **characterized in that** the organic solvent is selected from the hydrophilic organic solvents, the lipophilic organic solvents, the amphiphilic solvents and their mixtures.

**11.** Cosmetic composition according to Claim 10, **characterized in that** the organic solvent is isononyl isononanoate.

**12.** Cosmetic composition according to any one of Claims 9 to 11, **characterized in that** the photochromic coloring agent is located in the fatty phase, and the non-photochromic colouring matter is located in the aqueous phase.

**13.** Cosmetic composition according to any one of Claims 9 to 11, **characterized in that** the non-photochromic colouring matter is located in the fatty phase, and the photochromic coloring agent is located in the aqueous phase.

**14.** Composition according to any one of the preceding Claims, **characterized in that** it contains at least one additive selected from the preservatives, the fatty or aqueous phase thickeners, the perfumes, the lipophilic or hydrophilic active agents, the antioxidants, the colorants, the essential oils, the plant extracts, the vitamins and their derivatives, the sphingolipids, the liposoluble polymers in particular the hydrocarbons, and their mixtures.

**15.** Composition according to Claim 14, **characterized in that** the additive is selected from the polysaccharide biopolymers, the synthetic polymers, the hydrating agents, the UVA or B filters, the vitamins A, B, C and E, the ceramides, the polybutene, the polyalkenes, the polyacrylates and the silicone polymers compatible with the fatty bodies and their mixtures.

**16.** Composition according to any one of the preceding Claims, **characterized in that** it is available in the form of a product for the care and/or treatment and/or make-up of the skin and/or the hairs such as lipsticks, shimmer sticks, solid or liquid foundation creams, eyebrow pencils, eyeliners, mascaras, eye shadows, care creams, tinted or untinted and nail varnishes.

**17.** Use of the cosmetic composition such as defined according to any one of Claims 1 to 16 for the make-up of the skin and/or the hairs of the face and human body.

**18.** Cosmetic use of the composition such as defined according to any one of Claims 1 to 16 to care for and/or make-up and/or protect the skin and/or the hairs of the face and human body.

**19.** Use of the cosmetic composition such as defined according to any one of Claims 1 to 16 to care for and/or make-up and/or protect the skin and/or the hairs of the face and human body.

**20.** Use of the cosmetic composition such as defined according to any one of Claims 1 to 16 for the preparation of an ointment designed to treat and/or protect the skin and/or the hairs of the face and human body

**21.** Cosmetic treatment procedure for the skin and/or the hairs of the face and human body consisting of applying to the skin and/or the hairs a composition such as defined according to any one of the Claims 1 to 16.

**22.** Use in a cosmetic composition or for the manufacture of a coloured dermatological composition of a photochromic colouring agent and a non-photochromic colouring matter included in a cosmetic composition such as defined according to any one of Claims 1 to 16 in order to give a make-up return adapted to the environment.

**23.** Make-up procedure of the skin and/or the hairs of the face and the human body consisting of applying to the skin

and/or the hairs a cosmetic composition such as defined according to any one of Claims 1 to 16.

**Patentansprüche**

1. Flüssige kosmetische Zusammensetzung, in Form einer Emulsion, umfassend mindestens zwei verschiedene Phasen und enthaltend mindestens einen photochromen Farbstoff und mindestens einen nicht photochromen färbenden Stoff, **dadurch gekennzeichnet, dass** sich der (oder die) Farbstoff(e) der photochromen Färbung und die nicht photochrome(n) färbende(n) Substanz(en) in verschiedenen Phasen der Emulsion befinden.

2. Zusammensetzung nach Anspruch 1, **dadurch gekennzeichnet, dass** der photochrome Farbstoff ausgewählt ist aus photochromen Metalloxiden, Hydraten photochromer Metalloxide, Komplexen der Oxide oder der Hydrate photochromer Metalloxide und dotierten Aminosilicaten.

3. Zusammensetzung nach Anspruch 2, **dadurch gekennzeichnet, dass** der photochrome Farbstoff ein photochromes Metalloxid, ausgewählt aus Oxiden von Titan, Niob, Silizium, Aluminium, Zink, Hafnium, Thorium, Zinn, Thallium, Zirkonium, Beryllium, Kobalt, Calcium, Magnesium oder ein Hydrat dieser Oxide, ist.

4. Zusammensetzung nach einem der vorstehenden Ansprüche, **dadurch gekennzeichnet, dass** der photochrome Farbstoff 0,01 bis 30 % und bevorzugt 1 bis 16 Gew.-% darstellt, bezogen auf das Gesamtgewicht der Zusammensetzung.

5. Zusammensetzung nach einem der vorstehenden Ansprüche, **dadurch gekennzeichnet, dass** die nicht photochrome färbende Substanz ausgewählt ist aus lipophilen Farbstoffen, hydrophilen Farbstoffen, mineralischen und organischen Pigmenten, Perlmutten und ihren Mischungen.

6. Zusammensetzung nach Anspruch 5, **dadurch gekennzeichnet, dass** die nicht photochrome färbende Substanz ein Pigment ist, das in der Zusammensetzung im Verhältnis von 0 bis 5 Gew.-%, bevorzugt von 0,01 bis 5 Gew.-%, bezogen auf das Gesamtgewicht des Produkts vorhanden ist.

7. Zusammensetzung nach den Ansprüchen 5 oder 6, **dadurch gekennzeichnet, dass** die nicht photochrome färbende Substanz ein mineralisches Pigment, ausgewählt aus gelbem Eisenoxid, rotem Eisenoxid, schwarzem Eisenoxid und ihren Mischungen, ist.

8. Kosmetische Zusammensetzung nach einem der vorstehenden Ansprüche, **dadurch gekennzeichnet, dass** sie in Form einer Wasser-in-öl- (W/Ö), öl-in-wasser- (Ö/W) oder multiplen Emulsion vorliegt.

9. Zusammensetzung nach einem der vorstehenden Ansprüche, **dadurch gekennzeichnet, dass** mindestens eine der Phasen eine Fettphase, umfassend mindestens ein organisches Lösungsmittel, ist und die andere Phase eine wässrige Phase ist.

10. Kosmetische Zusammensetzung nach Anspruch 9, **dadurch gekennzeichnet, dass** das organische Lösungsmittel ausgewählt ist aus hydrophilen organischen Lösungsmitteln, lipophilen organischen Lösungsmitteln, amphiphilen Lösungsmitteln und ihren Mischungen.

11. Kosmetische Zusammensetzung nach Anspruch 10, **dadurch gekennzeichnet, dass** das organische Lösungsmittel Isononylisononanoat ist.

12. Kosmetische Zusammensetzung nach einem der Ansprüche 9 bis 11, **dadurch gekennzeichnet, dass** sich der photochrome Farbstoff in der Fettphase befindet und sich die nicht photochrome färbende Substanz in der wässrigen Phase befindet.

13. Kosmetische Zusammensetzung nach einem der Ansprüche 9 bis 11, **dadurch gekennzeichnet, dass** sich die nicht photochrome färbende Substanz in der Fettphase befindet und sich der photochrome Farbstoff in der wässrigen Phase befindet.

14. Zusammensetzung nach einem der vorstehenden Ansprüche, **dadurch gekennzeichnet, dass** sie mindestens einen Zusatzstoff enthält, der ausgewählt ist aus Konservierungsstoffen, Verdickungsmitteln der wässrigen oder

Fettphase, Parfümen, hydrophilen oder lipophilen Aktivstoffen, Antioxidantien, Farbstoffen, ätherischen Ölen, pflanzlichen Extrakten, Vitaminen und ihren Derivaten, Sphingolipiden, fettlöslichen Polymeren, insbesondere Kohlenwasserstoffe und ihre Mischungen.

**15.** Zusammensetzung nach Anspruch 14, **dadurch gekennzeichnet, dass** der Zusatzstoff ausgewählt ist aus polysaccharidischen Bioploymeren, synthetischen Polymeren, Feuchtigkeitsregulatoren, UVA- oder B-Filtern, Vitaminen A, B, C und E, Ceramiden, Polybuten, Polyalkylenen, Polyacrylaten und Sikiconpolymeren, verträglich mit den Fettkörpern und den Mischungen.

**16.** Zusammensetzung nach einem der vorstehenden Ansprüche, **dadurch gekennzeichnet, dass** sie in Form eines Produktes zur Pflege und/oder zur Behandlung und/oder zum Abdecken der Haut und/oder der Hautanhangsgebilde, wie Lippenstifte, Lip-Gloss, flüssige oder feste Grundierungen, Produkte gegen Augenschatten oder für die Augenumgebung, Eye-Liner, Wimperntuschen, Lidschatten, Pflegecremes, getönt oder nicht, und Nagellacke, vorliegt.

**17.** Verwendung der kosmetischen Zusammensetzung, wie nach einem der Ansprüche 1 bis 16 definiert, zum Abdecken der Haut und/oder der Hautanhangsgebilde des Gesichts und des menschlichen Körpers.

**18.** Kosmetische Verwendung der Zusammensetzung, wie nach einem der Ansprüche 1 bis 16 definiert, zum Pflegen und/oder Abdecken und/oder Schützen der Haut und/oder der Hautanhangsgebilde des Gesichts und des menschlichen Körpers.

**19.** Verwendung der kosmetischen Zusammensetzung, wie nach einem der Ansprüche 1 bis 16 definiert, zum Pflegen und/oder Abdecken und/oder Schützen der Haut und/oder der Hautanhangsgebilde des Gesichts und des menschlichen Körpers.

**20.** Verwendung der kosmetischen Zusammensetzung, wie nach einem der Ansprüche 1 bis 16 definiert, zur Zubereitung einer Salbe, bestimmt zum Behandeln und/oder Schützen der Haut und/oder der Hautanhangsgebilde des Gesichts und des menschlichen Körpers.

**21.** Verfahren zur kosmetischen Behandlung der Haut und/oder der Hautanhangsgebilde des Gesichts und des menschlichen Körpers, wobei man auf die Haut und/oder die Hautanhangsgebilde eine Zusammensetzung, wie nach einem der Ansprüche 1 bis 16 definiert, aufträgt.

**22.** Verwendung eines photochromen Farbstoffs und einer nicht photochromen färbenden Substanz, umfasst in einer kosmetischen Zusammensetzung, wie nach einem der Ansprüche 1 bis 16 definiert, in einer kosmetischen Zusammensetzung oder zur Herstellung einer dermatologischen, gefärbten Zusammensetzung um eine an die Umgebung angepasste Aufbringung einer Abdeckung zu ergeben.

**23.** Verfahren zum Abdecken der Haut und/oder der Hautanhangsgebilde des Gesichts und des menschlichen Körpers, wobei man auf die Haut und/oder die Hautanhangsgebilde eine kosmetische Zusammensetzung, wie nach einem der Ansprüche 1 bis 16 definiert, aufträgt.